# EUROPEAN PATENT SPECIFICATION

(11) **EP 2 798 051 B1**
(45) Date of publication and mention of the grant of the patent: **15.11.2017**
(21) Application number: 12810022.9
(22) Date of filing: 27.12.2012
(51) Int. Cl.: C10M 133/06, C10M 133/16, C07C 231/00, C07C 237/06, C07C 229/06

(54) **METHOD FOR PROVIDING LIMITED SLIP PERFORMANCE**
VERFAHREN ZUR BEREITSTELLUNG VERMINDERTER RUTSCHFÄHIGKEIT
PROCÉDÉ DE FOURNITURE DE EFFICACITE DE GLISSEMENT

(30) Priority: 29.12.2011 US 201161581182 P
(43) Date of publication of application: 05.11.2014
(73) Proprietor: The Lubrizol Corporation, Wickliffe, OH 44092-2298 (US)
(72) Inventor: BASU, Shubhamita, Wickliffe, OH 44092 (US); SACCOMANDO, Daniel J., Derby DE56 1QN (GB)
(74) Representative: D Young & Co LLP
(86) International application number: PCT/US2012/071731
(87) International publication number: WO 2013/101882

(56) References cited:
- WO-A1-2010/077630
- WO-A1-2010/096325
- WO-A1-2011/149810
- US-A- 3 048 620

## Description

### BACKGROUND OF THE INVENTION

The invention relates to a method of providing limited slip performance in a limited slip differential as defined in and by the appended claims.

A vehicle differential typically has bevel gear or spur gear planetary systems which distribute drive torque evenly to the two driving wheels irrespective of their rotational speed. This makes it possible for the driven wheels to roll during cornering without slip between the wheel and road surface in spite of their different rotational speed. When one wheel is on a low traction surface, the amount of torque that can be transmitted is limited to the amount which can be applied before the wheel slips.

A limited slip differential typically employs a wet multiplate clutch, i.e., clutch plates which are in contact with a lubricant, to supply more torque to the non-slipping wheels during a slipping situation. During normal turns, for one wheel to spin faster than the other, the clutch plates must disengage or break away. There can be NVH (noise, vibration, harshness) associated with this event.

In order for the slip to be controlled lubricants containing compounds capable of improving friction performance, dispersants and sulfur- and/or phosphorus- containing extreme pressure agents may be used. Examples of lubricants of this type are disclosed in US Patents 4,308,154; 4,180,466; 3,825,495; and European Patent Application 0 399 764 A1.

WO 2010/096325 to inventors Saccomando et al., published August 26, 2010, teaches a composition for use as a friction modifier for an automatic transmission, comprising a long chain hydrocarbyl amine having one or two additional groups on one or two different amine nitrogen atoms thereof. One of the species represented in the publication is shown in formula XIIa therein, having a formula of R¹N((CH₂)₂CONH(CH₂)₃NHR⁴)₂. The WO'325 publication does not teach the use of the hydrocarbyl amines for friction performance in limited slip differentials.

US 3,048,620 discloses tertiary-amino alkylated amide of the formula (H₂NCOCH₂CH₂)₂N-lower alkylene-N-(CH₂CH₂CONH₂)₂. WO 2011/149810 A1 discloses a method of lubricating a limited slip differential.

### SUMMARY OF THE INVENTION

An object of the present invention is to provide a method as disclosed herein that is capable of providing a high coefficient of friction and a low tendency toward noise, vibration and harshness (NVH) often manifested as chatter (i.e. an abnormal noise typically referred to as a low-frequency "growl" and "groan," particularly during low-speed cornering maneuvers). The inventors have discovered that, unexpectedly, the method disclosed herein may also be suitable for limited slip systems having one or more distinct plate materials. For example, the plate materials may be steel, paper, ceramic, carbon fibers and systems employing a mixture of plate types such as steel on ceramic, carbon fibers in paper or steel on paper.

The invention provides a method of providing limited slip performance in a limited slip differential comprising the step of introducing a lubricating composition comprising a major amount of an oil of lubricating viscosity, and an oil soluble compound of formula R'N((CH₂)₂CONHR")₂, wherein R' is H or a C₈₋₂₈ hydrocarbyl group, and wherein R" is H or a C₈₋₂₈ hydrocarbyl group, with the proviso that at least one of R' or R" is a hydrocarbyl group; and operating the limited slip differential.

We further provide a method of producing an oil soluble compound suitable for limited slip performance. The method comprises preparing a Michael adduct from an acrylate and an alkylamine of formula R'NH₂, followed by subjecting the Michael adduct to an amidation reaction with a further alkylamine of formula R"NH₂. R' and R" can be the same or different and are H or a C₈₋₂₈ hydrocarbyl group wherein up to 4 carbon atoms in the hydrocarbyl group may optionally be replaced by nitrogen or oxygen atoms.

### DETAILED DESCRIPTION OF THE INVENTION

Various preferred features and embodiments will be described below by way of non-limiting illustration.

As used herein the expression "oil-soluble" or "hydrocarbon soluble" is meant a material which will dissolve or disperse on a macroscopic or gross scale in an oil or hydrocarbon, as the case may be, typically a mineral oil, such that a practical solution or dispersion can be prepared. In order to prepare a useful lubricant formulation, the material should not precipitate or settle out over a course of several days or weeks. Such materials may exhibit true solubility on a molecular scale or may exist in the form of agglomerations of varying size or scale, provided however that they have dissolved or dispersed on a gross scale.

As used herein, the term "hydrocarbyl group" or "hydrocarbyl substituent" is used in its ordinary sense, which is well-known to those skilled in the art. Specifically, it refers to a group having a carbon atom directly attached to the remainder of the molecule and having predominantly hydrocarbon character. Examples of hydrocarbyl groups include:
(i) hydrocarbon substituents, that is, aliphatic (e.g., alkyl or alkenyl), alicyclic (e.g., cycloalkyl, cycloalkenyl) substituents, and aromatic-, aliphatic-, and alicyclic-substituted aromatic substituents, as well as cyclic substituents wherein the ring is completed through another portion of the molecule (e.g., two substituents together form a ring).

An oil-soluble compound used according to the present invention is a compound of formula R'N((CH₂)₂CONHR")₂, wherein R' and R" can be the same or different and are selected from H or a C₈₋₂₈ hydrocarbyl group, so long as the minimum amount of carbon atoms between R' and R" together is about C₈ or C₁₀.

In certain embodiments R' may be C₈₋₂₈, or a C₁₀-C₂₀, or a C₁₂-C₁₄ hydrocarbyl group. In a preferred embodiment, R' can
be a C₁₈ hydrocarbyl group such as an oleyl or stearyl group, a coco group, or -(CH₂)₃-NH-oleyl group, preferably oleyl. Similarly, in certain embodiments R" may be a C₈-C₂₈, or a C₁₀-C₂₀, or a C₁₂-C₁₄ hydrocarbyl group. In a preferred embodiment, R" can be an oleyl group, coco group, or -(CH₂)₃-NH-oleyl group, preferably oleyl. As used herein, oleyl group refers to 9-octadecene group and coco refers to cocoalkylamine or homologous amines. Cocoalkylamine (or cocoamine) is a primary amine containing predominantly C₁₂ & C₁₄ groups, derived from, derivable from, or characteristic of coconut oil.

Several preferred embodiments of the oil soluble compound, used in the invention, include, for example, oleyl-N((CH₂)₂CONH-oleyl)₂ (formula I), oleyl-N((CH₂)₂CONH-(CH2)₃-NH-oleyl)₂ (formula II), oleyl-N((CH₂)₂CONH-coco)₂ (formula III), and coco-N((CH₂)₂CONH-oleyl)₂ (formula IV).

While the oil soluble compounds may be produced according to various methods, an easy and cost-effective route discovered by the inventors encompasses the amidation of a Michael adduct.

A Michael adduct arises from a nucleophilic addition of a carbanion or other nucleophile to an α,β unsaturated carbonyl compound. A Michael adduct according to one aspect of the present invention may be prepared by reacting an acrylate of the formula CH₂=CHCOOR with an alkylamine of the formula R'NH₂, to form a Michael adduct of the formula R'N((CH₂)₂COOR)₂, wherein R can be H or a C₈₋₂₈ hydrocarbyl group, and R' is as described above. In a preferred embodiment, R can be CH₃. In the same or different embodiment, R' can be an oleyl group, coco group, or -(CH₂)₃-NH-oleyl group, preferably oleyl.

The Michael adduct can undergo an amidation reaction with an alkyl amine of the formula R"NH₂, wherein R" is as described above, to form the oil soluble compound of formula R'N((CH₂)₂CONHR")₂.

In one embodiment, the oil soluble compound of formula I can be produced by reacting methyl acrylate, that is, an acrylate of the formula CH₂=CHCOOCH₃, with oleylamine, that is, an alkylamine of the formula oleyl-NH₂, to form a Michael adduct of the formula oleyl-N((CH₂)₂COOCH₃)₂, and thereafter amidating the Michael adduct with an alkyl amine of the formula oleyl-NH₂, to form an oil-soluble compound of the formula oleyl-N((CH₂)₂CONH-oleyl)₂.

In another embodiment, the oil soluble compound of formula II can be produced by reacting methyl acrylate with oleylamine to form a Michael adduct of the formula oleyl-N((CH₂)₂COOCH₃)₂, and thereafter amidating the Michael adduct with an alkyl amine of the formula NH₂-(CH₂)₃-NH-oleyl to form an oil-soluble compound of the formula oleyl-N((CH₂)₂CONH-(CH₂)₃-NH-oleyl)₂.

In a further embodiment, the oil soluble compound of formula III can be produced by reacting methyl acrylate with oleylamine to form the Michael adduct, and thereafter amidating the Michael adduct with cocoamine, to form an oil-soluble compound of the formula oleyl-N((CH₂)₂CONH-coco)₂.

In an even further embodiment, the oil soluble compound of formula IV can be produced by reacting methyl acrylate with cocoamine to form the Michael adduct, and thereafter amidating the Michael adduct with oleylamine to form an oil-soluble compound of the formula coco-N((CH₂)₂CONH-oleyl)₂.

Oil-soluble compounds according to the foregoing embodiments can be employed in a lubricating composition with oils of lubricating viscosity to provide friction performance in limited slip differentials. The oil-soluble compounds can be included at a concentration of from about 0.1 to about 8 wt%, or 0.2 to about 7 wt%, and in some embodiments from about 0.5 to about 5 or about 6 wt%, or even from about 0.5 to about 3 or about 4 wt%.

### Oils of Lubricating Viscosity

The lubricating composition comprises an oil of lubricating viscosity. Such oils include natural and synthetic oils, oil derived from hydro cracking, hydrogenation, and hydrofinishing, unrefined, refined, re-refined oils or mixtures thereof. A more detailed description of unrefined, refined and re-refined oils is provided in International Publication WO2008/147704, paragraphs [0054] to [0056].

Synthetic lubricating oils include hydrocarbon oils and halo-substituted hydrocarbon oils such as polymerized and interpolymerized olefins, also known as polyalphaolefins; polyphenyls; alkylated diphenyl ethers; alkyl- or dialkylbenzenes; and alkylated diphenyl sulfides; and the derivatives, analogs and homologues thereof. Also included are alkylene oxide polymers and interpolymers and derivatives thereof, in which the terminal hydroxyl groups may have been modified by esterification or etherification. Also included are esters of dicarboxylic acids with a variety of alcohols, or esters made from C₅ to C₁₂ monocarboxylic acids and polyols or polyol ethers. Other synthetic oils include silicon-based oils, liquid esters of phosphorus-containing acids, and polymeric tetrahydrofurans. The synthetic oils may be produced by Fischer-Tropsch reactions and typically may comprise hydroisomerized Fischer-Tropsch hydrocarbons and/or waxes, or hydroisomerized slack waxes. In one embodiment oils may be prepared by a Fischer-Tropsch gas-to-liquid synthetic procedure as well as other gas-to-liquid oils.

Oils of lubricating viscosity may also be defined as specified in April 2008 version of "Appendix E - API Base Oil Interchangeability Guidelines for Passenger Car Motor Oils and Diesel Engine Oils", section 1.3 Sub-heading 1.3. "Base Stock Categories." In one embodiment, the oil of lubricating viscosity may be an API Group I, Group II, Group III, or Group IV oil.

Polyalphaolefins are categorized as Group IV oils. In one embodiment, at least 50% by weight of the oil of lubricating viscosity is a polyalphaolefin (PAO). Typically, the polyalphaolefins are derived from monomers having from 4 to 30, or from 4 to 20, or from 6 to 16 carbon atoms. Examples of useful PAOs include those derived from 1-decene. These PAOs may have a viscosity of 1.5 to 150 mm²/s (cSt) at 100°C. PAOs are typically hydrogenated materials.

The amount of the oil of lubricating viscosity present is typically the balance remaining after subtracting from 100 wt% the sum of the amount of the compound of the invention and the other performance additives.

The lubricating composition may be in the form of a concentrate and/or a fully formulated lubricant. If the lubricating composition of the invention (comprising the additives disclosed herein) is in the form of a concentrate which may be combined with additional oil to form, in whole or in part, a finished lubricant), the ratio of these additives to the oil of lubricating viscosity and/or to diluent oil includes the ranges of 1:99 to 99:1 by weight, or 80:20 to 10:90 by weight.

### Other Performance Additives

The composition used in the invention optionally further includes at least one other performance additive. The other performance additives include dispersants, metal deactivators, detergents, viscosity modifiers, extreme pressure agents (typically boron- and/or sulfur- and/or phosphorus-containing), antiwear agents, antioxidants (such as hindered phenols, aminic antioxidants or molybdenum compounds), corrosion inhibitors, foam inhibitors, demulsifiers, pour point depressants, seal swelling agents, friction modifiers and mixtures thereof.

The total combined amount of the other performance additives (excluding the viscosity modifiers) present on an oil free basis may include ranges of 0.0 wt% to 25 wt%, or 0.01 wt% to 20 wt%, or 0.1 wt% to 15 wt% or 0.5 wt% to 10 wt%, or 1 to 5 wt% of the composition. Although one or more of the other performance additives may be present, it is common for the other performance additives to be present in different amounts relative to each other.

In one embodiment the lubricating composition is free of molybdenum-containing additives.

### Viscosity Modifiers

In one embodiment, the lubricating composition further includes one or more viscosity modifiers. When present the viscosity modifier may be present in an amount of 0.5 wt% to 70 wt%, 1 wt% to 60 wt%, or 5 wt% to 50 wt%, or 10 wt% to 50 wt% of the lubricating composition.

Viscosity modifiers include (a) polymethacrylates, (b) esterified copolymers of (i) a vinyl aromatic monomer and (ii) an unsaturated carboxylic acid, anhydride, or derivatives thereof, (c) esterified interpolymers of (i) an alpha-olefin; and (ii) an unsaturated carboxylic acid, anhydride, or derivatives thereof, or (d) hydrogenated copolymers of styrene-butadiene, (e) ethylene-propylene copolymers, (f) polyisobutenes, (g) hydrogenated styrene-isoprene polymers, (h) hydrogenated isoprene polymers, or (i) mixtures thereof.

In one embodiment the viscosity modifier includes (a) a polymethacrylate, (b) an esterified copolymer of (i) a vinyl aromatic monomer; and (ii) an unsaturated carboxylic acid, anhydride, or derivatives thereof, (c) an esterified interpolymer of (i) an alpha-olefin; and (ii) an unsaturated carboxylic acid, anhydride, or derivatives thereof, or (d) mixtures thereof.

Dispersant viscosity modifiers (often referred to as DVMs) include functionalized polyolefins, for example, ethylene-propylene copolymers that have been functionalized with the reaction product of maleic anhydride and an amine, a polymethacrylate functionalized with an amine, or esterified styrene-maleic anhydride copolymers reacted with an amine may also be used in the composition of the invention.

### Extreme Pressure Agents

Extreme pressure agents include compounds containing boron and/or sulfur and/or phosphorus. The extreme pressure agent may be present in the lubricating composition at 0.0 wt% to 20 wt%, or 0.05 wt% to 10 wt%, or 0.1 wt% to 8 wt%, or 0.5 wt% to 6 wt% of the lubricating composition.

In one embodiment the extreme pressure agent is a sulfur-containing compound. In one embodiment the sulfur-containing compound may be a sulfurized olefin, a polysulfide, or mixtures thereof.

Examples of the sulfurized olefin include a sulfurized olefin derived from propylene, isobutylene, pentene; an organic sulfide and/or polysulfide including benzyldisulfide; bis-(chlorobenzyl) disulfide; dibutyl tetrasulfide; di-tertiary butyl polysulfide; and sulfurized methyl ester of oleic acid, a sulfurized alkylphenol, a sulfurized dipentene, a sulfurized terpene, a sulfurized Diels- Alder adduct, an alkyl sulfenyl N 'N-dialkyl dithiocarbamates; or mixtures thereof. In one embodiment the sulfurized olefin includes a sulfurized olefin derived from propylene, isobutylene, pentene or mixtures thereof.

In one embodiment, the extreme pressure agent sulfur-containing compound includes a dimercaptothiadiazole or derivative, or mixtures thereof. Examples of the dimercaptothiadiazole include 2,5-dimercapto-1,3,4-thiadiazole or a hydrocarbyl-substituted 2,5-dimercapto-1,3,4-thiadiazole, or oligomers thereof. The oligomers of hydrocarbyl-substituted 2,5-dimercapto-1,3,4-thiadiazole typically form by forming a sulfur-sulfur bond between 2,5-dimercapto-1,3,4-thiadiazole units to form derivatives or oligomers of two or more of said thiadiazole units. Suitable 2,5-dimercapto-1,3,4-thiadiazole derived compounds include 2,5-bis(tert-nonyldithio)-1,3,4-thiadiazole or 2-tert-nonyldithio-5-mercapto-1,3,4-thiadiazole.

The number of carbon atoms on the hydrocarbyl substituents of the hydrocarbyl-substituted 2,5-dimercapto-1,3,4-thiadiazole typically include 1 to 30, or 2 to 20, or 3 to 16.

In one embodiment the extreme pressure agent includes a boron-containing compound. The boron-containing compound includes a borate ester (which in some embodiments may also be referred to as a borated epoxide), a borated alcohol, a borated dispersant or mixtures thereof. In one embodiment the boron-containing compound may be a borate ester or a borated alcohol.

The borate ester may be prepared by the reaction of a boron compound and at least one compound selected from epoxy compounds, halohydrin compounds, epihalohydrin compounds, alcohols and mixtures thereof. The alcohols include dihydric alcohols, trihydric alcohols or higher alcohols, with the proviso for one embodiment that hydroxyl groups are on adjacent carbon atoms, i.e., vicinal.

Boron compounds suitable for preparing the borate ester include the various forms selected from the group consisting of boric acid (including metaboric acid, HBO₂, orthoboric acid, H3BO3, and tetraboric acid, H₂B₄O₇), boric oxide, boron trioxide and alkyl borates. The borate ester may also be prepared from boron halides.

In one embodiment suitable borate ester compounds include tripropyl borate, tributyl borate, tripentyl borate, trihexyl borate, triheptyl borate, trioctyl borate, trinonyl borate and tridecyl borate.

In one embodiment the borate ester compounds include tributyl borate, tri-2-ethylhexyl borate or mixtures thereof.

In one embodiment, the boron-containing compound is a borated dispersant, typically derived from an N-substituted long chain alkenyl succinimide. In one embodiment the borated dispersant includes a polyisobutylene succinimide. Borated dispersants are described in more detail in US Patents 3,087,936; and Patent 3,254,025.

In one embodiment the borated dispersant may be used in combination with a sulfur-containing compound or a borate ester.

In one embodiment the extreme pressure agent is other than a borated dispersant.

The number average molecular weight of the hydrocarbon from which the long chain alkenyl group was derived includes ranges of 350 to 5000, or 500 to 3000, or 550 to 1500. The long chain alkenyl group may have a number average molecular weight of 550, or 750, or 950 to 1000.

The N-substituted long chain alkenyl succinimides are borated using a variety of agents including boric acid (for example, metaboric acid, HBO₂, orthoboric acid, H₃BO₃, and tetraboric acid, H₂B₄O₇), boric oxide, boron trioxide, and alkyl borates. In one embodiment the borating agent is boric acid which may be used alone or in combination with other borating agents.

The borated dispersant may be prepared by blending the boron compound and the N-substituted long chain alkenyl succinimides and heating them at a suitable temperature, such as, 80 °C to 250 °C, or 90 °C to 230 °C, or 100 °C to 210 °C, until the desired reaction has occurred. The molar ratio of the boron compounds to the N-substituted long chain alkenyl succinimides may have ranges including 10:1 to 1:4, or 4:1 to 1:3; or the molar ratio of the boron compounds to the N-substituted long chain alkenyl succinimides may be 1:2.

An inert liquid may be used in performing the reaction to prepare the borated dispersant. The liquid may include oil, toluene, xylene, chlorobenzene, dimethylformamide or mixtures thereof.

In one embodiment the dispersant may be a post treated dispersant. The dispersant may be post treated with dimercaptothiadiazole, optionally in the presence of one or more of a phosphorus compound, a dicarboxylic acid of an aromatic compound, and a borating agent.

In one embodiment the post treated dispersant may be formed by heating an alkenyl succinimide or succinimide detergent with a phosphorus ester and water to partially hydrolyze the ester. The post-treated dispersant of this type is disclosed for example in U.S. Patent 5,164,103.

In one embodiment the post treated dispersant may be produced by preparing a mixture of a dispersant and a dimercaptothiadiazole and heating the mixture above about 100°C. The post treated dispersant of this type is disclosed for example in U.S. Patent 4,136,043.

In one embodiment the dispersant may be post treated to form a product prepared comprising heating together: (i) a dispersant (typically a succinimide), (ii) 2,5-dimercapto-1,3,4-thiadiazole or a hydrocarbyl-substituted 2,5-dimercapto-1,3,4-thiadiazole, or oligomers thereof, (iii) a borating agent (similar to those described above); and (iv) optionally a dicarboxylic acid of an aromatic compound selected from the group consisting of 1,3 diacids and 1,4 diacids (typically terephthalic acid), or (v) optionally a phosphorus acid compound (including either phosphoric acid or phosphorous acid), said heating being sufficient to provide a product of (i), (ii), (iii) and optionally (iv) or optionally (v), which is soluble in an oil of lubricating viscosity. The post treated dispersant of this type is disclosed for example in International Application WO 2006/654726 A.

Examples of a suitable dimercaptothiadiazole include 2,5-dimercapto-1,3,4-thiadiazole or a hydrocarbyl-substituted 2,5-dimercapto-1,3,4-thiadiazole. In several embodiments the number of carbon atoms on the hydrocarbyl-substituent group includes 1 to 30, 2 to 25, 4 to 20, or 6 to 16. Examples of suitable 2,5-bis(alkyl-dithio)-1,3,4-thiadiazoles include 2,5-bis(tert-octyldithio)-1,3,4-thiadiazole 2,5-bis(tert-nonyldithio)-1,3,4-thiadiazole, 2,5-bis(tert-decyldithio)-1,3,4-thiadiazole, 2,5-bis(tert-undecyldithio)-1,3,4-thiadiazole, 2,5-bis(tert-dodecyldithio)-1,3,4-thiadiazole, 2,5-bis(tert-tridecyldithio)-1,3,4-thiadiazole, 2,5-bis(tert-tetradecyldithio)-1,3,4-thiadiazole, 2,5-bis(tert-pentadecyldithio)-1,3,4-thiadiazole, 2,5-bis(tert-hexadecyldithio)-1,3,4-thiadiazole, 2,5-bis(tert-heptadecyldithio)-1,3,4-thiadiazole, 2,5-bis(tert-octadecyldithio)-1,3,4-thiadiazole, 2,5-bis(tert-nonadecyldithio)-1,3,4-thiadiazole or 2,5-bis(tert-eicosyldithio)-1,3,4-thiadiazole, or oligomers thereof.

Friction modifiers include fatty phosphonate esters, amine salts of phosphoric acid esters, reaction products from fatty carboxylic acids reacted with guanidine, aminoguanidine, urea or thiourea, and salts thereof, fatty amines, fatty hydroxyl amines, borated phospholipids, borates, borate esters, fatty phosphites, fatty acid amides, fatty epoxides, borated fatty epoxides, alkoxylated fatty amines, borated alkoxylated fatty amines, fatty poly ethers, metal salts of fatty acids, or fatty imidazolines, condensation products of carboxylic acids and poly alkylene-polyamines, fatty malimides, fatty tartrimides, and fatty oxazolines.

In one embodiment the lubricating composition may contain phosphorus- or sulfur- containing antiwear agents other than compounds described as an extreme pressure agent of the amine salt of a phosphoric acid ester described above. Examples of the antiwear agent may include a non-ionic phosphorus compound (typically compounds having phosphorus atoms with an oxidation state of +3 or +5), a metal dialkyldithiophosphate (typically zinc dialkyldithiophosphates), a metal mono- or di- alkylphosphate (typically zinc phosphates), or mixtures thereof.

The non-ionic phosphorus compound includes a phosphite ester, a phosphate ester, or mixtures thereof. A more detailed description of the non-ionic phosphorus compound include column 9, line 48 to column 11, line 8 of US 6,103,673.

In one embodiment the lubricating composition of the invention further includes a dispersant. The dispersant may be a succinimide dispersant (for example N-substituted long chain alkenyl succinimides), a Mannich dispersant, an ester-containing dispersant, a condensation product of a fatty hydrocarbyl monocarboxylic acylating agent with an amine or ammonia, an alkyl amino phenol dispersant, a hydrocarbyl-amine dispersant, a polyether dispersant or a poly ether amine dispersant.

In one embodiment the succinimide dispersant includes a polyisobutylene-substituted succinimide, wherein the polyisobutylene from which the dispersant is derived may have a number average molecular weight of 400 to 5000, or 950 to 1600.

Succinimide dispersants and their methods of preparation are more fully described in US Patents 3,172,892, 3,219,666, 3,316,177, 3,340,281, 3,351,552, 3,381,022, 3,433,744, 3,444,170, 3,467,668, 3,501,405, 3,542,680, 3,576,743, 3,632,511, 4,234,435, Re 26,433, and 6,165,235, 7,238,650 and EP Patent Application 0 355 895 A.

Suitable ester-containing dispersants are typically high molecular weight esters. These materials are described in more detail in U.S. Patent 3,381,022.

In one embodiment the dispersant includes a borated dispersant.

Typically the borated dispersant includes a succinimide dispersant including a polyisobutylene succinimide, wherein the polyisobutylene from which the dispersant is derived may have a number average molecular weight of 400 to 5000. Borated dispersants are described in more detail above within the extreme pressure agent description.

Corrosion inhibitors include amines, fatty amines, 1-amino-2-propanol, octylamine octanoate, condensation products of dodecenyl succinic acid or anhydride and/or a fatty acid such as oleic acid with a polyamine.

Metal deactivators include derivatives of benzotriazoles (typically tolyltriazole),1,2,4-triazoles, benzimidazoles, 2-alkyldithiobenzimidazoles, thiadiazoles, or 2-alkyldithiobenzothiazoles. The metal deactivators may also be described as corrosion inhibitors.

Foam inhibitors include copolymers of ethyl acrylate and 2-ethylhexyl acrylate, and optionally vinyl acetate. Also included are siloxanes, typically polydimethylsiloxanes

Demulsifiers include trialkyl phosphates, and various polymers and copolymers of ethylene glycol, ethylene oxide, propylene oxide, or mixtures thereof.

Pour point depressants include esters of maleic anhydride-styrene, polymethacrylates, polyacrylates or polyacrylamides.

Seal swell agents include Exxon Necton-37™ (FN 1380) and Exxon_Mineral Seal Oil™ (FN 3200).

### Industrial Application

The self-contained lubricant of the limited slip differential is generally different from the lubricant supplied to a manual transmission or an automatic transmission fluid.

An axle gear may have any one of a number of different types of differentials. A differential typically has three major functions. The first function is to transmit engine power to the wheels. The second function is act as the final gear reduction in the vehicle, slowing the rotational speed from the transmission to the wheels. The third function is to transmit the power to the wheels while allowing them to rotate at different speeds. A number of differentials are known and include an open differential, a clutch-type limited slip differential, a viscous coupling differential, a Torsen differential and a locking differential. All of these differentials may be generically referred to as axle gears.

Axle gears typically require a lubricant. The lubricant formulation is dependent on the type of axle gear, and the operating conditions of the axle gear. For example, an open differential axle gear is believed to require antiwear and/or extreme pressure additives. A limited slip differential further requires a friction modifier because, in addition to an open differential (known from many axle fluids), a spring pack and a clutch pack are typically present. The clutch pack may contain one or more reaction plates (often made from steel) and one or more friction plates. The friction plates are known, and may be made from a number of materials including paper, carbon, graphite, steel and a composite.

The lubricating composition suitable for the limited slip differential may have a sulfur content in the range of 0.3 wt% to 5 wt%, or 0.5 wt% to 5 wt%, or 0.5 wt% to 3 wt% or 0.8 wt% to 2.5 wt%, or 1 wt% to 2 wt%.

In one embodiment the lubricating composition suitable for the limited slip differential may be a fully formulated fluid.

In one embodiment the lubricating composition suitable for the limited slip differential may be a top treat concentrate.

When the lubricating composition is in the form of a top treat concentrate, the concentrate may be added at 0.2 wt% to 10 wt%, or 0.5 wt% to 7 wt%, 0.5 wt% to 5%, or 1.0 to 3.0 wt% relative to the amount of lubricant in a limited slip differential.

In an embodiment of the invention, a method of providing limited slip performance is provided comprising introducing a lubricating composition as disclosed herein to a differential, and operating the differential.

The lubricant composition and method disclosed herein may be suitable for limited slip systems having one or more distinct plate materials. For example the plate materials may be steel, paper, ceramic, carbon fibers and systems employing a mixture of plate types such as steel on ceramic, carbon fibers in paper or steel on paper.

The amount of each chemical component described is presented exclusive of any solvent or diluent oil, which may be customarily present in the commercial material, that is, on an active chemical basis, unless otherwise indicated. However, unless otherwise indicated, each chemical or composition referred to herein should be interpreted as being a commercial grade material which may contain the isomers, by-products, derivatives, and other such materials which are normally understood to be present in the commercial grade.

It is known that some of the materials described above may interact in the final formulation, so that the components of the final formulation may be different from those that are initially added. For instance, metal ions (of, e.g., a detergent) can migrate to other acidic or anionic sites of other molecules. The products formed thereby, including the products formed upon employing the composition of the present invention in its intended use, may not be susceptible of easy description. Nevertheless, all such modifications and reaction products are included within the scope of the present invention; the present invention encompasses the composition prepared by admixing the components described above.

### EXAMPLES

Preparative Example 1 (EX1): Oleyl amine (152.5g) and methanol (350g) are charged to a 1L 4-necked flask. Methyl acrylate (97.8g) is added drop-wise via an addition funnel at room temperature and the reaction is heated. Excess methanol is removed under reduced pressure and additional oleyl amine (303g) is added. The reaction mixture is heated until 80% of the theoretical distillate was collected and the rest was removed under vacuum. The final product is an off-white waxy solid at room temperature.

Preparative Example 2 (EX2): Oleyl amine (152.5g) and methanol (350g) are charged to a 1L 4-necked flask. Methyl acrylate (98g) is added drop-wise via an addition funnel at room temperature and the reaction is heated. Excess methanol is removed under reduced pressure and NH₂-(CH₂)₃-NH-oleyl amine (365g) is added. The reaction mixture is heated until 80% of the theoretical distillate was collected and the rest was removed under vacuum. The final product is an off-white waxy solid at room temperature.

Preparative Example 3 (EX3): Oleyl amine (294g) and methanol (700g) are charged to a 2L 4-necked flask. Methyl acrylate (189g) is added drop-wise via an addition funnel at room temperature over 45 minutes and the reaction is heated. Excess methanol is removed under reduced pressure and coco amine (779.5g) is added. The reaction mixture is heated until 80% of the theoretical distillate was collected and the rest was removed under vacuum. The final product is an off-white waxy solid at room temperature.

Preparative Example 4 (EX4): Coco amine (281.2g) and methanol (750g) are charged to a 3L 4-necked flask. Methyl acrylate (238g) is added drop-wise via an addition funnel at room temperature over 1 hour, and the reaction mixture is heated. Excess methanol is removed under pressure. Oleyl amine (698.2g) is added and the reaction mixture is heated until 80% of the theoretical distillate was collected and the rest was removed under vacuum. The final product is an off-white waxy solid at room temperature.

### Axle Lubricants

Comparative Example 1 (CE1) is a commercially available axle fluid having the formulation in table 1, and containing no additional limited slip friction modifier.

| Table 1 | |
|---|---|
| Component | Wt% - active basis |
| Oil of lubricating viscosity | 65.00 |
| Extreme Pressure Agent | 5.38 |
| Viscosity Modifier | 25.06 |
| Corrosion Inhibitor | 0.22 |
| Antiwear | 1.66 |
| Dispersant | 2.41 |
| Antifoam | 0.15 |
| Friction modifier | 0.13 |

Comparative Example 2 (CE2) is a commercially available axle fluid having the formulation in table 2 and containing 1.8 wt % of a commercially available phosphorus-containing friction modifier.

| Table 2 | |
|---|---|
| Component | Wt% - active basis |
| CE1 | 95.0 |
| Further oil of lubricating viscosity | 2.2 |
| Phosphorous containing FM | 1.8 |

Axle Lubricant 1 (ALEX1) is CE1 axle fluid top-treated with 1.8 wt % of EX1.

Axle Lubricant 2 (ALEX2) is CE1 axle fluid top-treated with 1.8 wt % of EX2.

Axle Lubricant 3 (ALEX3) is CE1 axle fluid top-treated with 1.8 wt % of the product of EX3.

Axle Lubricant 4 (ALEX4) is CE1 axle fluid top-treated with 1.8 wt % of the product of EX4.

Lubricants for testing are prepared by adding one of the materials from the preparative examples identified in the tables below to the indicated base formulation. The lubricants containing EX1 to EX4 are evaluated in a Full-Scale Low-Velocity Friction Apparatus (FSLVFA). The apparatus uses a clutch test specimen as defined by SAE Paper 2010-01-2231. The test is run while varying the speed, temperature and pressure. The test consists of friction performance evaluations at the beginning and after a 17-hour durability stage. A break-in phase runs 10 minutes at 90 °C oil temperature, 16 rpm, and 7070 N load. The phase conditions the clutch system for the pre-durability performance evaluation. The pre-durability performance evaluation is achieved by ramping the speed from 0 to 5 rpm in 5 seconds, then back to zero. Load is set to two levels, 3535 N and 7070 N, which correspond to the range of axial compressive load imposed by the axle's internal clutch pack. The above two loads are evaluated at three oil temperatures: 40 °C, 90 °C, and 120 °C. The sample clutch pack undergoes a durability phase that involves running the test rig for 17 hours at 120 °C oil temperature, 7070 N load, and 16 rpm. The post-durability evaluation is then run using the same conditions as the pre-test evaluation. A more detailed description of the test procedure is provided in SAE Paper 2010-01-2231. The table below shows a post-durability rating of NVH (@5 rpm) and curvature. The data obtained is as follows:

| Lubricant | Pre Test Evaluation | | Post Test Evaluation | |
|---|---|---|---|---|
| | Torque (N-m) | Torque vs Time Graph Shape | Torque (N-m) | Torque vs Time Graph Shape |
| CE1 | 37 | Smooth, linear | Range 31-42 | oscillating |
| CE2 | 34 | Smooth, linear | 31 | Smooth, linear |
| ALEX1 | 35 | Smooth, linear | 28 | Smooth, linear |
| ALEX2 | 35 | Smooth, linear | 25 | Smooth, linear |

| Fluid | NVH (@ 5 rpm) | Curvature |
|---|---|---|
| CE1 | 2.1 | 7.6 |
| CE2 | 0.6 | 4.4 |
| ALEX1 | 0.7 | 3.0 |
| ALEX2 | 0.6 | 0.6 |

| | | |
|---|---|---|
| Footnotes: Noise, Vibration, Harshness (NVH) at 5 rpm is the standard deviation of the torque signal based upon a moving average of the torque signal during the 2 second hold at 5 rpm. A high NVH rating is indicative of the occurrence of "stick-slip" at the friction surface. Torque spikes when the plates "stick" and drops when the plates "slip". NVH describes the amplitude of the torque signal and is independent of the shape of the torque signal. Good FM candidates should have low NVH at 5 rpm. Curvature describes the shape of the torque signal which is believed to be related to the difference between the static and dynamic friction coefficients. Curvature is the average difference between the torque when the plates breakaway and come to rest versus the torque during the 2 second hold at 5 rpms. A positive curvature means the torque signal is concave down during the sweep (bows downward). A negative curvature means the torque signal is concave up (bows upward) during the sweep. Ideally curvature should be close to zero which would mean the torque signal is flat across all speeds. Slight negative curvature value is acceptable but high positive curvature value is less desirable. | | |

The mention of any document is not an admission that such document qualifies as prior art or constitutes the general knowledge of the skilled person in any jurisdiction. Except in the Examples, or where otherwise explicitly indicated, all numerical quantities in this description specifying amounts of materials, reaction conditions, molecular weights, number of carbon atoms, and the like, are to be understood as modified by the word "about."

It is to be understood that the upper and lower amount, range, and ratio limits set forth herein may be independently combined. Similarly, the ranges and amounts for each element of the invention can be used together with ranges or amounts for any of the other elements.

## Claims

1. A method of providing limited slip performance in a limited slip differential comprising the step of introducing a lubricating composition comprising a major amount of an oil of lubricating viscosity, and an oil soluble compound of formula R'N((CH₂)₂CONHR")₂, wherein R' is H or a C₈₋₂₈ hydrocarbyl group, and wherein R" is H or a C₈₋₂₈ hydrocarbyl group, with the proviso that at least one of R' or R" is a hydrocarbyl group; and operating the limited slip differential.

2. The method of claim 1 wherein R' and R" are C₁₈ hydrocarbyl groups.

3. The method of claim 2, wherein R' and R" are both oleyl.

4. The method of claim 1, wherein R' is oleyl, and R" is coco.

5. The method of claim 1, wherein R' is coco, and R" is oleyl.

## Patentansprüche

1. Verfahren zur Bereitstellung von Teilsperrungsverhalten in einem Sperrdifferential mit Teilsperrung, umfassend den Schritt des Einbringens einer Schmiermittelzusammensetzung, die eine überwiegende Menge eines Öls mit einer Schmierölviskosität und eine öllösliche Verbindung mit der Formel R'N((CH₂)₂CONHR")₂ umfasst, wobei R' H oder eine Kohlenwasserstoffgruppe mit 8 bis 28 C-Atomen ist und wobei R" H oder eine Kohlenwasserstoffgruppe mit 8 bis 28 C-Atomen ist, unter der Bedingung, dass R' und/oder R" eine Kohlenwasserstoffgruppe ist; und des Betreibens des Sperrdifferentials mit Teilsperrung.

2. Verfahren nach Anspruch 1, wobei R' und R" Kohlenwasserstoffgruppen mit 18 C-Atomen sind.

3. Verfahren nach Anspruch 2, wobei sowohl R' als auch R" Oleyl sind.

4. Verfahren nach Anspruch 1, wobei R' Oleyl ist und R" Kokos ist.

5. Verfahren nach Anspruch 1, wobei R' Kokos ist und R" Oleyl ist.

## Revendications

1. Procédé consistant à fournir une performance de glissement limité dans un différentiel à glissement limité comprenant l'étape consistant à introduire une composition lubrifiante comprenant une quantité majoritaire d'une huile de viscosité lubrifiante et un composé soluble dans l'huile de formule R'N((CH₂)₂CONHR")₂, R' étant H ou un groupe hydrocarbyle en C₈₋₂₈ et R" étant H ou un groupe hydrocarbyle en C₈₋₂₈, à condition qu'au moins l'un de R' ou R" soit un groupe hydrocarbyle ; et faire fonctionner le différentiel à glissement limité.

2. Procédé selon la revendication 1, R' et R" étant des groupes hydrocarbyle en C₁₈.

3. Procédé selon la revendication 2, R' et R" étant tous deux des groupes oléyle.

4. Procédé selon la revendication 1, R' étant un groupe oléyle et R" étant un groupe coco.

5. Procédé selon la revendication 1, R' étant un groupe coco et R" étant un groupe oléyle.
